# EUROPEAN PATENT APPLICATION

(11) **EP 4 552 687 A1**
(43) Date of publication of application: **14.05.2025**
(21) Application number: 23209361.7
(22) Date of filing: 13.11.2023
(51) Int. Cl.: A61N 5/06, A61C 15/00

(54) **PERSONAL CARE DEVICE**

(71) Applicant: Koninklijke Philips N.V., 5656 AG Eindhoven (NL)
(72) Inventor: GOTTENBOS, Bart, Eindhoven (NL)
(74) Representative: Philips Intellectual Property & Standards

(57) **Abstract**

Disclosed concepts provide a personal care device for illuminating a surface of a user with light. Specifically, a light emitting element (for emission of light toward the surface of the user when in use) is attached to a body of the personal care device via a mounting arrangement. The mounting arrangement is configured such that, when in use, the light emitting element is able to move relative to the body. Accordingly, the light emitting element may be provided closer to the surface of the user when in use, and so may better illuminate certain parts of the surface of the user. In particular, the light emitting element may be provided close/within crevasses of the surface of the user (e.g., interdental spaces) to provide better levels of illumination.

## Description

### FIELD OF THE INVENTION

The present invention relates to a personal care device for illuminating a surface of a user with light.

### BACKGROUND OF THE INVENTION

There is an increasing trend of including light emitting elements in personal care devices for providing a therapeutic effect for the user.

In terms of oral health applications, blue to violet light has been shown to inhibit the growth of oral biofilms, thus reducing dental plaque. Additionally, blue light can also reduce the inflammation response of human gingival cells, thus directly modulating gingival inflammation without even reducing plaque. Furthermore, red or even infrared light has been shown to be valuable for treating certain skin and musculoskeletal conditions, for wound healing, and to stimulate hair growth.

To improve the efficacy of such devices, a number and/or density of the light emitting elements are typically increased. In addition, a longer exposure time may be recommended to ensure that a measurable therapeutic effect arises (e.g., providing the technology in a brushing mouthpiece rather than a toothbrush to allow for larger application times). However, such solutions result in increased costs and/or additional inconvenience.

Accordingly, there exists a need for a personal care device capable of providing treatment by one or more light emitting elements with improved efficacy.

### SUMMARY OF THE INVENTION

According to examples in accordance with an aspect of the invention, there is provided a personal care device.

The personal care device comprises a body for placement proximate to a surface of a user in use;
a light emitting element configured to emit light toward the surface of the user in use; and
a mounting arrangement configured to attach the light emitting element to the body,
wherein, when in use, the mounting arrangement is configured to enable movement of the light emitting element relative to the body.

Disclosed concepts provide a personal care device for illuminating a surface of a user with light. Specifically, a light emitting element (for emission of light toward the surface of the user when in use) is attached to a body of the personal care device via a mounting arrangement. The mounting arrangement is configured such that, when in use, the light emitting element is able to move relative to the body. Accordingly, the light emitting element may be provided closer to the surface of the user when in use, and so may better illuminate certain parts of the surface of the user. In particular, the light emitting element may be provided close/within crevasses of the surface of the user (e.g., interdental spaces) to provide better levels of illumination.

Put another way, a personal care device according to the invention provides a mounting of the light emitting element in such a way that the light emitting element may be provided nearer to the surface of the user (e.g., into interdental spaces). This is achieved by mounting the light emitting elements so that they may (at least) move up-and-down relative to the body of the device, rather than being fixedly mounted on the device.

The body of the personal care device is the support structure to which the light emitting element is mounted. For example, if the personal care device is a toothbrush, the body may be considered to be the bristle platen. If the personal care device is a mouthpiece, the body may be considered to be the mouthpiece plane. That is, the body is not the element of the device that the user directly handles. In essence, the body is an element of the personal care device that is positioned near the part of the surface of the user to which a personal care function is being applied.

The mounting arrangement mechanically connects the light emitting element to the body of the personal care device, thus providing a single device. However, the mounting element is adapted such that the light emitting element may flexibly move relative to the body. As a result, should the light emitting element come into contact with the surface of the user while in use, the light emitting element may follow the contour of the surface of the user, providing light closer to the surface of the user.

In contrast, light emitting elements on existing personal care devices are fixedly/statically mounted to the body of the personal care device. Accordingly, the light emitting element is located a certain distance from the surface of the user. The light intensity from a light emitting element decreases with distance (due to a dispersion angle). Therefore, a distant surface may not receive an adequate light intensity. This problem is compounded when the surface is irregular (e.g., due to wrinkles, or due to interdental spaces), as such surfaces may not receive adequate light levels, compounded by shadow effects. Moreover, these irregular surfaces may be at the greatest need of light treatment (e.g., due to build up of microbes).

As a result, by attaching the light emitting elements in such a way that relative movement to the body is enabled, the light emitting element may provide light closer to the surface of the user, enabling illumination of surfaces that would otherwise receive inadequate dosages of light.

In some embodiments, the mounting arrangement may comprise a biasing element configured to bias the light emitting element away from the body.

Accordingly, the light emitting element is provided as far away from the body as allowed by the mounting arrangement while still attached to the body, in a passive manner. By being resiliently biased, the light emitting element is pushed toward the surface of the user, even as the distance between the surface of the user and the body increases (up to the maximum distance between light emitting element and the body enabled by the mounting arrangement).

For example, the body may be placed proximate a tooth of the user. As a result, the light emitting element may be provided in contact with the tooth. When the body is moved to be proximate an interdental space (e.g., a surface that is further away from the body than the tooth surface), the light emitting element may be biased into the interdental space by virtue of the biasing element forcing the light emitting element away from the body without having to force the body closer to the surface of the user.

Specifically, the biasing element may comprise a spring structure resiliently biasing the light emitting element away from the body. For example, the spring structure may comprise a coil spring or a leaf spring.

Alternatively or additionally, the mounting arrangement may comprise an actuator configured to selectively position the light emitting element relative to the body.

This active positioning of the light emitting element may enable increased control and precision of the position of the light emitting element, and therefore improved efficacy. In particular, this may be beneficial to adapt the distance of the light emitting element if, for example, the light emitting element contacting the surface would be less effective (e.g., for a more comfortable treatment). However, this may be more expensive and complicated compared to the passive biasing option.

When in use, the mounting arrangement may be configured to enable movement of the light emitting element in a direction substantially parallel to the body.

By enabling movement parallel to the body, the light emitting element may stay in indentations/crevasses (e.g., interdental spaces, wrinkles, nasal cavities, etc.) for a longer period of time while the device is moved over the surface of the user. For example, if the body is moved back-and-forth over an interdental cavity with a relatively small amplitude (e.g., during brushing), the light emitting element may remain in the interdental space throughout.

Also, when in use, the mounting arrangement may be configured to enable movement of the light emitting element in a direction substantially perpendicular to the body.

This ensures that the light emitting element may be provided as close as possible to/within an indentation/crevasse, whilst also ensuring that discomfort is not felt (e.g., due to force exerted in the direction of the surface.

A wavelength of the emitted light may be in a range of 350nm to 1mm.

Light of various wavelengths in this range may provide different treatments and therapeutic benefits. For example, red or infrared light may be valuable for certain skin treatments or can reduce pain or inflammation in oral health on gums. Light in an UV range may be useful for anti-microbial purposes at low intensities.

More particularly, the wavelength of the emitted light may be in a range of 400nm to 460nm.

This wavelength range may be of particular interest, for example, for oral health application. Indeed, this light has been shown to be useful for inhibiting growth of oral biofilms (thereby reducing dental plaque), as well as reducing the inflammation response of human gingival cells.

In some embodiments, the personal care device may be an oral care device.

For example, the personal care device may be a toothbrush, or a mouthpiece. In this case, the body may be a toothbrush platen, or a mouthpiece plane.

This may be of particular interest for the invention as there exist many undulating surfaces and indentations in the teeth that may not be properly exposed to light from light emitting elements that are fixed to the body. Indeed, interdental spaces often facilitates the growth of biofilms and production of plaque. Therefore, delivery of adequate light levels to said interdental spaces is of particular interest. By mounting the light emitting elements such that they may move relative to the body (rather than being fixed on the toothbrush platen or mouthpiece plane), improved illumination of these areas may be achieved.

In this case, the light emitting element may be shaped to at least partially fit into an interdental space of the user.

Not only is it beneficial to mount the light emitting element such that they may be provided in contact/near the interdental spaces of the user, but it may also be beneficial to enable the light emitting elements to enter the interdental spaces to ensure adequate illumination of such spaces.

This may be achieved by shaping the light emitting element to be a negative of the interdental space. Thus, the light emitting element may be wedge shaped or pyramid shaped.

In some embodiments, the light emitting element may have a maximum width of less than 3mm. This may ensure that the light emitting element can at least partially enter spaces less than 3mm in width, to better illuminate such spaces. If less than 0.5mm, the light emitting element may enter interdental spaces entirely.

More particularly, the light emitting element may have a maximum width in a range of 1-2mm. This may ensure that the light emitting element may allow the positioning of the light emitting element at an interdental space entrance, whilst avoiding complete entry and potential for discomfort and harm to gums.

The light emitting element may comprise an integrated light source.

That is, the light emitting element may generate the light itself, and thereby emit light toward the surface of the user.

Alternatively, the personal care device may further comprise a light source configured to generate the light for emission by the light emitting element, the light source provided separately from the light emitting element; and a light guide configured to transfer the light generated by the light source to the light emitting element.

That is, the light source may be provided as a separate element of the personal care device (e.g., generated in the handle, or in the body), and then the light is guided to the light emitting element for emission to the surface of the user via a light/waveguide. This may mean that the light source may be larger than the light emitting element. In addition, if multiple light emitting elements are provided, only a single light source may be required. Furthermore, the generation of light often results in the generation of heat also, which may be undesirable or even prejudicial to safety of the user if generated at the light emitting element contacting the surface of the user.

In either case, the light source may be an LED or a laser.

Many alternative sources of light may be used. Nevertheless, LEDs may be simple and cheap to implement in the personal care device, while lasers may generate with the benefit of directionality and having a thin wavelength band.

Furthermore, the light emitting element may comprise a lens arrangement configured to focus light on the surface of the user in use. This may increase the amount of light incident on the surface of the user, and thus improve efficacy of the personal care device.

Alternatively, the lens arrangement may be configured to disperse light to the surface of the user in use. In this way an illumination area may be widened. This may be useful when the light emitting element is within a space/cavity to steer light to opposing proximal surfaces. In particular, this would ensure illumination of all surfaces when the light emitting element is moved deeper into a space/cavity rather than over a flat surface.

In addition, the personal care device may comprise a plurality of light emitting elements configured to emit light toward the surface of the user in use. In this case, the mounting arrangement may be configured to attach each of the light emitting elements to the body, and when in use the mounting arrangement may be configured to enable movement of each of the light emitting elements relative to the body.

Of course, in some cases it may be desired to have multiple light emitting elements to ensure adequate dosages of light provided by the personal care device.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents simplified schematic diagrams of a personal care device for emitting light toward a surface of the user according to the prior art;
Fig. 2 presents a simplified schematic diagram of a personal care device in the form of a mouthpiece according to an embodiment of the invention; and
Fig. 3 presents a simplified schematic diagram of a personal care device in use according to another embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Disclosed concepts provide a personal care device for illuminating a surface of a user with light. Specifically, a light emitting element (for emission of light toward the surface of the user when in use) is attached to a body of the personal care device via a mounting arrangement. The mounting arrangement is configured such that, when in use, the light emitting element is able to move relative to the body. Accordingly, the light emitting element may be provided closer to the surface of the user when in use, and so may better illuminate certain parts of the surface of the user. In particular, the light emitting element may be provided close/within crevasses of the surface of the user (e.g., interdental spaces) to provide better levels of illumination.

Light treatment can be beneficial for treating a wide variety of conditions. It has been shown that light of certain wavelengths can be used to treat some skin and musculoskeletal conditions, for wound healing and inflammation reduction, to stimulate hair growth, and to inhibit growth of biofilms. Of particular interest is the use of blue and violet light to reduce plaque levels and gum inflammation.

Specifically, blue to violet light (400-460nm) can inhibit the growth of oral biofilms, thereby reducing dental plaque. Additionally, blue light can also reduce the inflammation response of human gingival cells, in this way directly modulating gingival inflammation without needing to reduce/remove plaque. Such technology may be best employed in mouthpieces to facilitate longer application times for larger light doses and thus improved efficacy.

It has now been realized that many of the conditions targeted by the use of light arise where the surface of the user undulates. In other words, the targeted conditions (wounds, biofilms, etc.) often develop in crevasses, indents, cavities, and similar spaces. For example, many oral health problems begin in interdental spaces (e.g., gingivitis on the interdental papilla or interproximal caries). It is therefore key to expose such locations to an adequate dosage of light in order to produce the advantages associated with such treatment.

However, in existing personal care devices, light emitting elements providing such treatment and therapeutic effects are placed at a certain distance from the surface of the user by virtue of being fixed to a body of the personal care device. For example, the light emitting element may be provided on a platen of a toothbrush, a plane of a mouthpiece, or a support structure of a facemask. From that position, the light emitting elements illuminate the surface of the user. Spaces (e.g., crevasses, indents, cavities, and similar spaces of the surface of the user) that are further away from the light emitting element fixed to the body of the personal care device may not receive adequate amounts of light. Indeed, light from light emitting elements tend to disperse with an increasing distance receive smaller amounts of light.

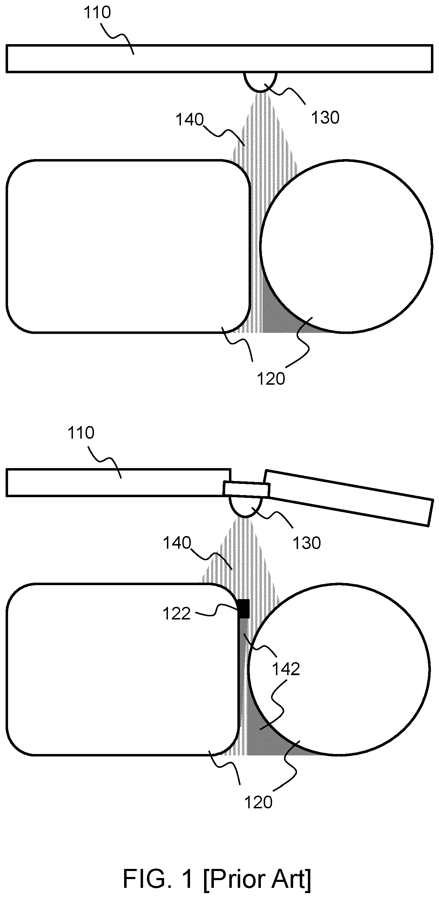

The above problem is demonstrated in Fig. 1. This shows a light emitting element 130 fixed on a body 110 of a personal care device. The body 110 is positioned over a surface 120 of a user, and in this case where there exists a cavity/undulation in the surface 120 of the user. This cavity may be, for example, an interdental space. The body 110 may not be able to be positioned closer to the surface 120 of the user without causing discomfort to the user, for example due to additional elements such as bristles, or the due to the overall shape of the body 110, not shown here for simplicity.

As seen, the light 140 emitted by the light emitting element 130 disperses (i.e., spreads out with distance). Accordingly, the surface 120 of the user in the cavity may not receive the same dosage of light 140 as the upper flat surface 120 of the user, or even an adequate dosage of light 140. As a result, a treatment or therapeutic effect expected to be affected by the light 140 may not occur. Furthermore, there may even be shadowed 142 areas in the cavity, receiving no direct light.

More particularly, when the personal care device is used for oral care, as light emitting element 130 has an emitting angle, the light intensity decreases with distance. Therefore, interdental spaces receive the lowest light dose, rendering such personal care devices ineffective for treatment of the interdental spaces. This is further hampered by the light rays from a distant light emitting element 130 being incident to the surface 120 at shallow angles in the interdental space, creating large shadow effects 142 and thus reducing light intensity.

This problem may be compounded by other factors, as shown in the lower diagram. For example, a piece of debris 122 may cause a shadowing effect 142, meaning that much of the surface 120 of the user in the cavity may not receive any direct light 140. For example, the debris 122 may be plaque built up in an interdental space of a user. Even when the body is flexible (as shown in the lower diagram), thus altering an angle of the emitted light 140, these issues may arise.

Accordingly, areas of the surface of the user that are most prone to various issues receive the least amount of light. Embodiments of the invention aim to overcome this issue by mounting the light emitters in such a way that they adapt their distance to the body of the personal care device to follow the contour of the surface of the user. Accordingly, a light intensity in the cavities, wrinkles, interdental spaces etc. is improved by providing the light emitting element closer to such regressed surfaces.

When the personal care device is an oral care device, embodiments aim to maximize the light intensity in interdental spaces and other distant tooth locations. At the same time, it may also remove or circumvent toothpaste on the oral surface which blocks the light, which may be an issue in particular where the light treatment is performed during tooth brushing (e.g., where the personal care device is a light emitting brushing mouthpiece).

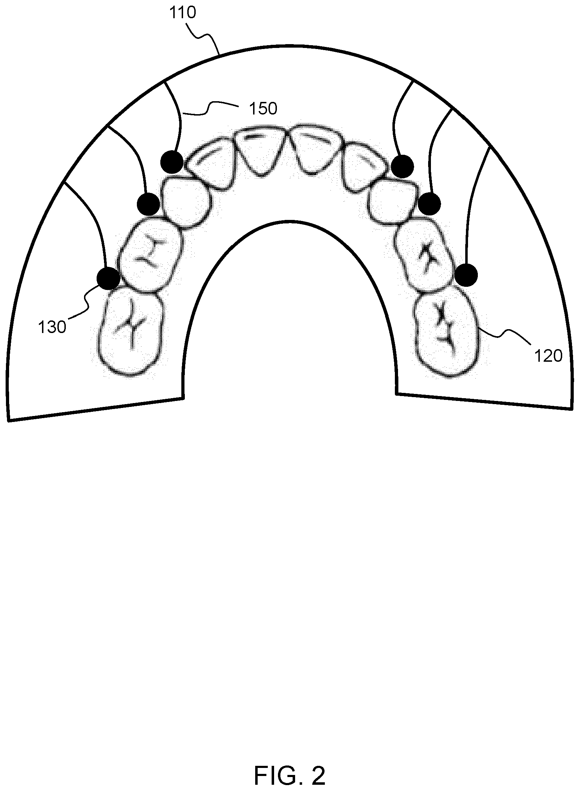

As shown in Fig. 2, embodiments of the invention may provide light emitting elements 130 mounted adaptively on the body 110 of the personal care device in such a way that they are as close as possible to the target treatment area (typically in contact). In other words, light emitting elements 130 are attached to the body 110 by a mounting assembly 150 comprising flexible arms that can easily bend back and forth, in this way following the contour of the teeth.

In general, embodiments of the invention attach the light emitting element(s) to the body of the personal care device whilst enabling/facilitating movement of the light emitting element(s) relative to the body. The movement is enabled in a direction substantially perpendicular to the body (i.e., toward and away from the body, or away and toward the surface of the user). The movement may additionally or alternatively be enabled in a direction substantially parallel to the body (e.g., side-to-side).

That is, not only does the mounting arrangement allow movement in the direction from the body of the personal care device to the surface of the user (e.g., by being flexible in such a direction), but also allows movement in other directions (e.g., by being flexible in those directions), most notably parallel to the body, which will be a typical user motion. That is, the light emitting elements may not directly/immediately follow the movement in the direction that the user moves the body of the personal care across the surface of the user. This allows the light emitting element(s) to stay longer in cavities, wrinkles, interdental spaces, etc., in this way additionally increasing the received local light dose.

Thus, embodiments of the invention may give rise to the following advantages:
(i) Improved light intensity in indents, cavities, wrinkles, interdental spaces and other locations distant from the body of the personal care device (e.g., the gum line);
(ii) Steeper impact angles of the light rays on the proximal surfaces, reducing the occurrence of shadow effects;
(iii) Negating or circumventing the negative impact of debris on the surface of the user (e.g., toothpaste, sweat, dirt, etc.) by virtue of the light emitting element potentially being in contact with the surface (removing the debris, or simply pushing past the debris); and
(iv) Increased light dosage in intensity in indents, cavities, wrinkles, interdental spaces through longer contact time (when movement substantially parallel to the body is enabled).

In an exemplary embodiment, the personal care device is a dental/oral care appliance (e.g. a cleaning mouthpiece, a light treatment tray or a toothbrush) with one or more light emitting elements (e.g. LEDs, Lasers, or the light emitting surface of a light guide). The mounting of the light emitting element to the oral care device is such that a distance between the light emitting element and a body (e.g., toothbrush platen, mouthpiece plane) of the oral care device is variable. This enables the light emitting element to follow the contour of the target surface. At a surface on a wide part of the tooth, the light emitting element will be closer to the oral care device, and at the interdental spaces the light emitting element will be further away. In some embodiments, the light emitting element may be inserted in the interdental space.

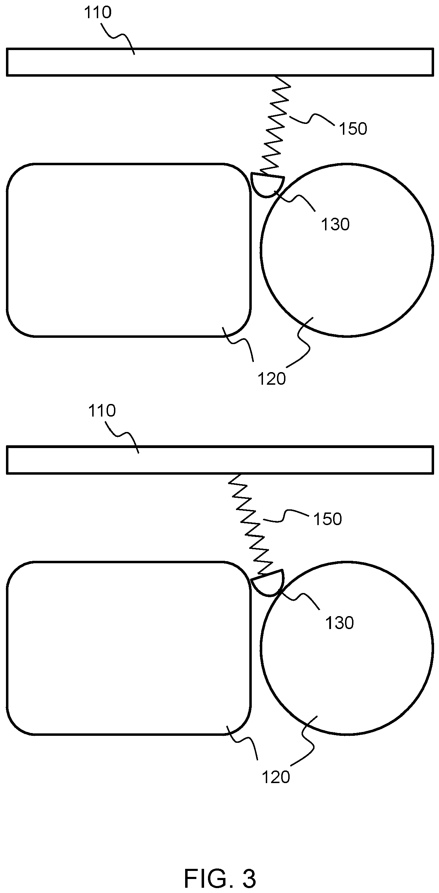

In general, personal care devices according to embodiments of the invention comprise the following components, as shown in Fig. 3:
(i) a body 110;
(ii) one or more light emitting elements 130; and
(iii) a mounting arrangement 150 configured to attach the light emitting element(s) 130 to the body 110.

The body 110 is the support structure that is positioned near the target surface 120 of the user. This is not the handle of the personal care device (e.g., handle of the toothbrush), but the part of the personal care device that is provided near to the surface 120 of the user to which a personal care function is to be applied. For example, the body 110 may be a head of a toothbrush/toothbrush platen, a plane of the mouthpiece, a helmet structure of a hair growth device, or another structure held over a surface 120 of the subject. The body 110 may be otherwise referred to as a shell or a support structure that is provided near to/proximate the surface 120 of the subject when in use.

The body 110 may be flexible, but may not come into complete contact with the surface 120 of the subject during (regular/normal) use of the personal care device. For example, a head of a toothbrush will not (typically) come into contact with the oral surface 120 of the subject, but the bristles attached to the head will. A plane of a mouthpiece may come into contact with the oral surface 120 in a few locations, but may generally be situated apart from the surface 120 of the user. A helmet for inducing growth of hair may come into contact with the head of the user, but in most will sit just above the head of the user.

In existing devices the one or more light emitting elements 130 are attached to the body 110 in a fixed manner, such that they are generally provided apart from the surface 120 of the user when in use, simply irradiating light from the location of the body 110.

Each light emitting element 130 is configured to emit light toward the surface 120 of the user in use. A wavelength of the emitted light may be in a range of 350nm to 1mm. Red and infrared light may be valuable for certain skin treatments and may reduce pain or inflammation of gums. Blue and violet light is particularly suitable for anti-microbial applications. UV light may also be applied for this application, but may be unsafe if used at high intensities. Preferably, for oral applications, a wavelength of the emitted light may be in a range of 400nm to 460nm.

Any light source may be used to produce the light for emission toward the surface 120 of the subject, such as a laser or an LED.

For example, the light emitting element 130 may comprise an integrated light source. That is, the light source may be implemented in the form of an LED or laser attached to the body 110 by the mounting arrangement 150. In that case the mounting arrangement 150 may also have embedded electrical wires to power the LED. An LED would typically be embedded in a transparent plastic or elastomer to make a watertight assembly. A part of the watertight assembly may form a lens to direct the light toward the surface 120 of the user when in use.

The light source may however also be positioned elsewhere in the personal care device (e.g., in the body 110, or on a handle of the device). In this case, the light may be transported via a light guide through the mounting arrangement 150. In this case, the surface 120 where the light is emitted would be the light emitting element 130.

In other words, the personal care device may comprise a light source provided separately from the light emitting element 130, and configured to generate the light for emission by the light emitting element 130, the light source. In this case a light guide is provided and configured to transfer the light generated by the light source to the light emitting element 130. The light guide may be provided in the mounting arrangement 150, or as a separate part to the lighting arrangement.

In either case, the light emitting element 130 may comprise a lens arrangement configured to focus light on the surface 120 of the user in use. The lens arrangement directs light that may otherwise be scattered/dispersed to other parts of the surface 120 and surroundings to a targeted part of the surface 120.

Alternatively, the lens arrangement may disperse light to the surface of the user. In this way an illumination area may be widened. This may be useful when the light emitting element is within a space/cavity to steer light to opposing proximal surfaces. In particular, this would ensure illumination of all surfaces when the light emitting element is moved deeper into a space/cavity rather than over a flat surface.

Overall, different types of lenses of the lens arrangement may be used to manipulate the light output angle and direction from the light emitting element.

The lens arrangement may be provided on a light emitting part of the light source, if the light source is integrated in the light emitting element 130. Alternatively, the lens arrangement may be provided on a light exit surface 120 of the light guide when the light source is provided separately from the light emitting element 130.

To enable the light emitting element 130 to (at least partially enter) various cavities, indentations, undulations of the surface 120 of the subject, the shape and dimensions of the light emitting element 130 (including any surrounding casing, etc.) should be carefully selected.

For example, in an oral care application, the light emitting element 130 may be shaped to (at least partially) fit into an interdental space of the user. To achieve this, the light emitting element 130 may be an approximate negative of a general/average interdental space. Therefore, the light emitting element 130 may be wedge shaped or three-sided pyramid shaped. Furthermore, if the approximate position of the light emitting element 130 on the dentition is known (e.g. on a mouthpiece the posterior or anterior position) its shape could even be more tailored to a general/average negative of the interdental space in that specific position.

Moreover, the light emitting element 130 and any surrounding material should be small enough to fit into a crevasse of the surface 120 of the user. It is not necessary for the light emitting element 130 to be so small that it can fully enter the space completely. Indeed, this may not be preferable from a user experience perspective, as when the light emitting element 130 is so small to fully enter a space it may cause discomfort.

Generally, it would be preferable for the light emitting element 130 to have a maximum width of less than 3mm. In general, an ideal range of a maximum width would be between 1mm and 2mm. For oral health applications, a light emitting element 130 of such a size may partially enter the interdental space without causing discomfort to gums and other surrounding oral tissue.

Moving on, and as shown in Fig. 3, the mounting arrangement 150 is configured to attach the light emitting element 130 to the body 110. When in use, the mounting arrangement 150 is configured to enable movement of the light emitting element 130 relative to the body 110, as shown by the difference in the upper to lower figure.

Generally, the mounting arrangement 150 pushes/biases the light emitting element 130 toward a target area of the surface 120 of the subject (e.g. an interdental space, wound, hair follicle site, etc.). In other words, the mounting arrangement 150 may bias the light emitting element 130 away from the body 110.

As shown in Fig. 3, one solution for such a biasing/pushing action is to use a biasing element including a spring construction. That is, the biasing element may comprise a spring structure resiliently biasing the light emitting element 130 away from the body 110. In such a case, if the user urges the body 110 of the personal care device toward the surface 120 (e.g., biting down on a mouthpiece, positioning a helmet, etc.), the light emitting element(s) 130 are pushed toward the body 110 of the device by the surface 120 of the user. In this way, a light emitting element 130 remains in contact with the surface 120 as the user moves the body 110 of the device over the surface 120, and the light emitting element 130 follows the contour of the surface 120, getting as deep as physically possible into any surface 120 indent.

In Fig. 3, the adaptive connection is drawn as bending rib like structures (e.g. leaf springs), but spiral springs may offer even better adaptability additionally in a direction parallel to the plane of the body 110 (i.e. a sideways direction). Adding sideway adaptability is beneficial for allowing the light emitting element 130 to remain in the indent for a longer period of time. If the user oscillates the body 110 back and forth (as shown by the difference between the top and bottom diagram) with a relatively small amplitude (e.g. 5-10mm) the light emitting element 130 may stay near to the interdental spaces during the oscillation.

When forcing the body 110 of the device toward the surface 120, the mounting arrangement 150 having a biasing element will be loaded. To excessive bending of the spring upon applying such a force, (which could compromise the spring loading action) the mounting arrangement 150 could be designed to behave stiff in the forcing direction.

Of course, there are many other ways of mounting the light emitting element 130 adaptively to the body 110 of the device. Generally, the mounting arrangement 150 may comprise passive spring like structures. However, the mounting arrangement 150 may be an active element (i.e., driven by an actuator). The active element/actuator could be configured to selectively position the light emitting element 130 relative to the body 110. This would be more complicated and expensive, but could offer the advantage to adapt the distance of the light emitting element 130. This may be beneficial when noncontact positions of the light emitting element 130 would be more beneficial (e.g. for a more comfortable treatment).

In general, the mounting arrangement 150 is configured to enable movement of the light emitting element 130 in a direction substantially parallel and/or perpendicular to the body 110. This enables the light emitting element 130 to be provided, and remain, in a location proximate to various indentations (e.g., interdental spaces, wrinkles, etc). Such indentations may not typically receive adequate dosages of light, despite an apparent need. Therefore, the proposed personal care device may be more effective than a personal acre device having light emitting elements 130 fixed to a body 110 of the personal care device.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope. The block diagrams in the Figures illustrate the architecture, functionality, and operation of possible implementations of systems, methods, and computer program products according to various embodiments of the present invention. In this regard, each block in the block diagrams may represent a module, segment, or portion of instructions, which comprises one or more executable instructions for implementing the specified logical function(s). It will also be noted that each block of the block diagrams, and combinations of blocks in the block diagrams, can be implemented by special purpose hardware-based systems that perform the specified functions or acts or carry out combinations of special purpose hardware and computer instructions.

## Claims

1. A personal care device, comprising:
a body (110) for placement proximate to a surface (120) of a user in use;
a light emitting element (130) configured to emit light (140) toward the surface of the user in use; and
a mounting arrangement (150) configured to attach the light emitting element to the body, wherein, when in use, the mounting arrangement is configured to enable movement of the light emitting element relative to the body.

2. The personal care device of claim 1, wherein the mounting arrangement (150) comprises a biasing element configured to bias the light emitting element (130) away from the body (110).

3. The personal care device of claim 2, wherein the biasing element comprises a spring structure resiliently biasing the light emitting element (130) away from the body (110), and optionally wherein the spring structure comprises a coil spring or a leaf spring.

4. The personal care device of any of claims 1-3, wherein the mounting arrangement (150) comprises an actuator configured to selectively position the light emitting element (30) relative to the body (110).

5. The personal care device of any of claims 1-4, wherein, when in use, the mounting arrangement (150) is configured to enable movement of the light emitting element (130) in a direction substantially parallel to the body (110).

6. The personal care device of any of claims 1-5, wherein, when in use, the mounting arrangement (150) is configured to enable movement of the light emitting element (130) in a direction substantially perpendicular to the body (110).

7. The personal care device of any of claims 1-6, wherein a wavelength of the emitted light is in a range of 350nm to 1mm, and preferably in a range of 400nm to 460nm.

8. The personal care device of any of claims 1-7, wherein the personal care device is an oral care device.

9. The personal care device of claim 8, wherein the light emitting element (130) is shaped to at least partially fit into an interdental space of the user, and preferably wherein the light emitting element is wedge or pyramid shaped.

10. The personal care device of any of claims 1-9, wherein the light emitting element (130) has a maximum width of less than 3mm, and preferably wherein the light emitting element has a maximum width in a range of 1-2mm.

11. The personal care device of any of claims 1-10, wherein the light emitting element (130) comprises an integrated light source.

12. The personal care device of any of claims 1-10, further comprising:
a light source configured to generate the light (140) for emission by the light emitting element (130), the light source provided separately from the light emitting element; and
a light guide configured to transfer the light generated by the light source to the light emitting element.

13. The personal care device of claim 11 or 12, wherein the light source is an LED or a laser.

14. The personal care device of any of claims 1-13, wherein the light emitting element (130) comprises a lens arrangement configured to focus light (140) on the surface of the user in use.

15. The personal care device of any of claims 1-14, further comprising a plurality of light emitting elements (130) configured to emit light (140) toward the surface (120) of the user in use, wherein
the mounting arrangement (150) is configured to attach each of the light emitting elements to the body (110), and when in use the mounting arrangement is configured to enable movement of each of the light emitting elements relative to the body.
